# EUROPEAN PATENT SPECIFICATION

(11) **EP 2 274 026 B1**
(45) Date of publication and mention of the grant of the patent: **16.03.2022**
(21) Application number: 09727638.0
(22) Date of filing: 02.04.2009
(51) Int. Cl.: A61M 1/06, A61J 13/00

(54) **HANDS-FREE BREAST PUMP SYSTEM**
HANDFREIES BRUSTPUMPENSYSTEM
SYSTÈME DE TIRE-LAIT MAINS LIBRES

(30) Priority: 03.04.2008 US 42095 P
(43) Date of publication of application: 19.01.2011
(73) Proprietor: Brittner, Lyndon, Provo, UT 84603 (US)
(72) Inventor: Brittner, Lyndon, Provo, UT 84603 (US)
(74) Representative: Kordel, Mattias
(86) International application number: PCT/US2009/039335
(87) International publication number: WO 2009/124199

(56) References cited:
- US-A- 4 680 028
- US-A- 4 857 051
- US-A- 4 964 851
- US-A- 5 049 126
- US-A- 5 071 403
- US-A1- 2002 198 489
- US-A1- 2006 106 334

## Description

### 1. The Field of the Invention

The present invention relates to a hands-free breast pump system and a method of hands-free collection of milk.

### 2. State of the Art

Studies show that mother's breast milk is more healthy for infants than formula or other types of milk. Mothers strive to provide the best environment for their children. Sometimes this requires that the mother cannot be with a nursing infant at all times. For example, some women work some amount of time during the day. Nursing mothers that work must collect milk during the day to provide breast milk for her child when she is away from her child. If a nursing mother does not pump, her milk production can wane, such that she is not able to produce enough milk for her infant. Thus, many working mothers collect breast milk to be able to work and provide the best nourishment for their infants. To accommodate nursing mothers, breast pumps for expressing breast milk for later use by her infant have been around for some time.

Typically, these breast pumps include a funnel, or parabolic-shaped cup, similar to a suction cup, which is placed over the nipple and a portion of the breast. The cup is generally connected to a container for holding the expressed milk and a vacuum pump of some type. Some pumps may be hand-activated, while others are electrically operated. Some are even battery powered.

A vacuum from the pump is generally intermittently generated within the shield to generate negative pressure on the nipple, causing milk to be expressed from the breast within the cup. The intermittent nature of the vacuum may be done to simulate a baby sucking at the breast for milk. The expressed milk then generally flows from the shield to a storage container for later use. Most breast pumps require that the woman use her hands to operate the pump and/or maintain connection with the cup and her breast. Such breast pumps have been time consuming and somewhat awkward to use because the woman using the pump must occupy her hands, making it difficult or impossible to perform other activities.

A variety of breast pumps have been developed that are intended to allow a woman's hands to be free during use of the breast pump. US 2006/106334 A1 discloses a hands-free breast pump device, which comprises a breast shield having an inner surface configured to form a seal with a lactating woman's breast, and an adapter configured to couple the breast shield to a breast pump system. The breast pump device comprises a fluid container for receiving breast milk. Often, these breast pumps utilize straps, or bra-type structures for holding the shield in the place during milk expression. However, these straps and other structures generally provide for additional bulk in the breast pump and are difficult and time consuming to attach, which is not conducive to pumping in locations other than home where the pump may be stored. Some women desire to be out of the house during times when she would need to pump breast milk to maintain milk production. Other pumps require special bras or other clothing, requiring often uncomfortable choices in clothing. Similarly, many breast pumps on the market are uncomfortable, and difficult to use. Thus, a need exists for a simple, comfortable, hands-free breast pump. It is therefore an object of the invention to provide a simpler and more comfortable hands-free breast pump device as well as a related method of hands-free collection of milk.

### SUMMARY OF THE INVENTION

This object is achieved by providing a hands-free breast pump device having the features of appended claim 1, as well as by providing a method for hands-free collection of milk as defined in appended claim 13. Preferable embodiments are disclosed in the dependent claims.

Embodiments of hands-free breast pump systems, methods, and components are described. The breast pump device includes a formed member, or breast shield, made of a material that provides for an adhesive inner surface for adhering to a woman's breast. The adhesiveness of the surface is due to the materials used during manufacturing, and not due to adhesive sprays, lotions, or other items placed on the breast shield or the breast by the end-user.

The breast pump device includes an adapter connected to the breast shield for transferring a vacuum generated by a pump to the breast to express milk. The adapter also allows milk expressed from the breast to drain from the adapter to a container, without travelling into the pump. The breast shield adheres to the breast and supports the weight of the adapter, breast shield, and tubing extending from the adaptor without separate adhesives, gels, straps, or specially designed support bras. Thus, the adhesive breast shield and breast pump system may allow for hand-free expression of milk.

### BRIEF DESCRIPTION OF THE DRAWINGS

Various examples and embodiments are shown and described in reference to the numbered drawings wherein:
FIG. 1 illustrates a generalized schematic view of an example of a breast pump system not comprised by the invention;
FIG. 2 illustrates a generalized schematic view of an exemplary embodiment of a breast pump system;
FIG. 3 illustrates a partial assembly of an exemplary breast pump system;
FIG. 4 illustrates components of a partial assembly of an exemplary breast pump system;
FIG. 5 illustrates a partial assembly of an exemplary breast pump system;
FIG. 6 illustrates a cross-sectional view of a lid assembly of an exemplary breast pump system;
FIGS. 7 through 9 illustrate views of exemplary breast shields of exemplary embodiments of a breast pump system.

Together with the following description, the Figures demonstrate and explain the principles of patient positioning systems and associated components and methods. In the Figures, the thickness and configuration of components may be exaggerated for clarity. The same reference numerals in different Figures represent the same component.

### DETAILED DESCRIPTION

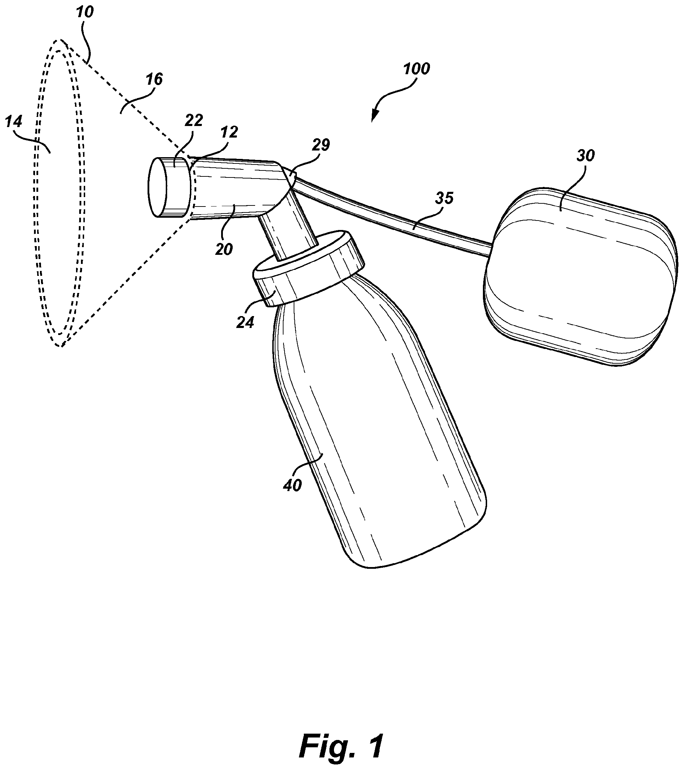

Examples and embodiments of a hands-free breast pump system are described below and shown in the Figures. Breast pump system 100, as shown in Fig. 1, includes breast shield 10, adapter 20, pump 30, vacuum line 35, vacuum line connector 29, container connector 24, and container 40.

Breast shield 10 may have outer surface 16 and an inner surface 14. Breast shield 10 may be made of a soft flexible material capable of conforming to a woman's breast. Inner surface 14 may be adhesive in nature so that breast shield 10 will adhere to a woman's breast. Because of the adherent nature of inner surface 14 of breast shield 10, inner surface 14 may attract dirt, lint, skin cells, oil, and other materials that may reduce the adhesiveness of inner surface 14. In that situation, inner surface 14 may be washed with soap and water, boiled, or otherwise cleaned to restore the adhesiveness of inner surface 14. Overtime, breast shield 10 may become worn, lose some adhesion properties, or otherwise require replacement. In such cases, breast shield 10 may be removed from adaptor 20 and replaced as necessary.

The adhesive nature of inner surface 14 of breast shield 10 may allow breast shield 10 to remain affixed to a woman's breast during the duration required to express a required or desired amount of milk or until dry. Thus breast shield 10 may be used in a hands-free manner without the need for separate consumer applied adhesives, gels, straps, or specialty bras designed for holding a breast pump system in place. Similarly, it allows use of breast pump system 100 without requiring the woman to hold breast shield 10 in place with her hands. Therefore, a woman using breast shield 10 with pump system 100 may be able to express milk and still have use of her hands for other activities. Additionally, breast shield 10 may be more comfortable than previously known breast shields because it conforms to the breast.

Breast shield 10 may be made from any appropriate material that imparts the desired attributes of flexibility and adhesiveness to skin. In certain embodiments, breast shield 10 may be made from an elastomeric material that has been sufficiently plasticized along inner surface 14 to provide the desired material characteristics. For example, breast shield 10 may be made from a silicone rubber with suitable plasticizers. In other examples, breast shield 10 may be made from Styrene-Ethylene-Butylene-Styrene (SEBS), Styrene-Ethylene-Propylene-Styrene (SEPS), and Styrene-Ethylene-Ethylene-Propylene-Styrene (SEEPS) copolymers. Other materials may also be appropriate. For example, suitable plasticizers for elastomers may include oils such as mineral oils, resins, rosins, and others. Other components may be used with the elastomers as well, such as antioxidants, colorants, bleed reducing additives, etc. In some embodiments, a coating may be applied during manufacture to provide the necessary adhesion properties. Depending on the desired structure, rigidity, softness, etc., any suitable process or materials may be used to construct breast shield 10, as desired. For example, in some instances it may be desirable to have more or less rigidity than others.

The material used in forming breast shield 10 may be manufactured by solvent blending, melt blending, or compounding under heat and pressure such as by use of a single screw or twin screw compounding machine or otherwise. Breast shield 10 may be constructed by injection molding, casting, or another desired process.

Breast shield 10 may be configured in any shape and dimension compatible with a woman's breast, as desired. For example, some embodiments of breast shield 10 may be funnel-shaped or cup-shaped. It should be understood that breast shield 10 may be produced and marketed in a number of sizes and shapes in order to be compatible with a wide range of breast dimensions, profiles, and shapes. Breast shield 10 may include opening 12 for connecting breast shield 10 to connector sleeve 22 of adapter 20. Breast shield 10 may also be manufactured to work with known breast pump systems.

In some embodiments, breast shield 10 may be able to invert, such that inner surface 14 is temporarily on the outside and outer surface 16 is temporarily on the inside. By inverting breast shield 10 a woman using breast pump system 100 may be able to achieve a tighter, more secure fit. A woman may first place opening 22 over the nipple in a desired position, and then extending or rolling breast shield 10 over the breast as breast shield is returned to the normal state, ensuring maximum contact, fit, and adhesion between breast shield 10 and the breast.

Turning now to adapter 20, embodiments of adapter 20 may provide for introducing a vacuum to the woman's breast and for directing the flow of milk to container 40. Adapter 20 may include a connector for connecting to breast shield 10. For example, adapter 20 may include connector sleeve 22 that forms an interference connection with opening 12 and inner surface 14 of breast shield 10. Opening 12 may be stretched around sleeve 22 to form the interference fit. The end of sleeve 22 may be configured to seal against or around the areola of a breast. Sleeve 22 may also be configured so that the nipple of the breast extends inside sleeve 22. Inner surface 14 may be configured to adhere to the skin of the breast surrounding the areola.

In some embodiments, sleeve 22 may be generally flush with opening 12. For example, sleeve 22 may include a groove or lip on or near the edge extending into breast shield 10 for holding the inside of opening 12. Similarly, opening 12 of breast shield 10 may include a complimentary structure to allow coupling of sleeve 22 and breast shield 10. In other embodiments, adapter 20 and breast shield 10 may be a unitary structure.

Adapter 20 may include connector 24 for connecting adaptor 20 to container 40. For example, connector 24 may be threaded to engage threads on container 40. Adapter 20 may be configured such that milk drawn into adapter 20 drains into container 40, without going into pump 30. Container 40 may be any container used for receiving expressed milk or a modification thereof. For example, container 40 may be a standard baby bottle, or other container commonly used to store and/or deliver milk to an infant.

Adapter 20 may be connected through vacuum line connector 29 to pump 30 via vacuum line 35. Negative pressure generated by pump 30 may be transmitted to adapter 20 via vacuum line 35 and thereby to the interior of breast shield 10 and sleeve 22. Pump 30 may be any pump or device suitable for delivering vacuum pressure sufficient for expressing milk. Vacuum line 35 may be made of any material capable of transferring negative pressure from pump 30 to adapter 20, and may be any desired configuration. For example, vacuum line 35 may be plastic tubing, such as Polyvinyl Chloride (PVC) tubing.

Vacuum line 35 may be connected to pump 30 and vacuum line connector 29 of adapter 20 via any type of connector desired. For example, vacuum line connector 29 may include an opening about the same diameter or slightly smaller that the outer diameter of vacuum line 35, providing for a press or interference fit of the outside of vacuum line into adaptor 20. Similarly, vacuum line connector 29 may include an inner flange for an appropriate fit with the inner surface of vacuum line 35. Similarly, vacuum line connector 29 may be oriented in any desired direction from connector 20, depending on the desired location of pump 30. For example, vacuum line connector 29 may be oriented such that vacuum line 35 extends collinearly with drain line 45 to minimize the profile of adaptor 20 when attached to a breast.

In some embodiments, adapter 20 may be specially designed to meet the functional requirements described herein. In any of the embodiments, it may be desirable to have adapter 20 be as small as possible to reduce the weight of adapter 20. Similarly, adaptor 20 may be made of light materials to reduce the weight being born by breast shield 10, and the woman's breast. Adaptor 20 may also be manufactured to be compatible with any desired commercially available pump.

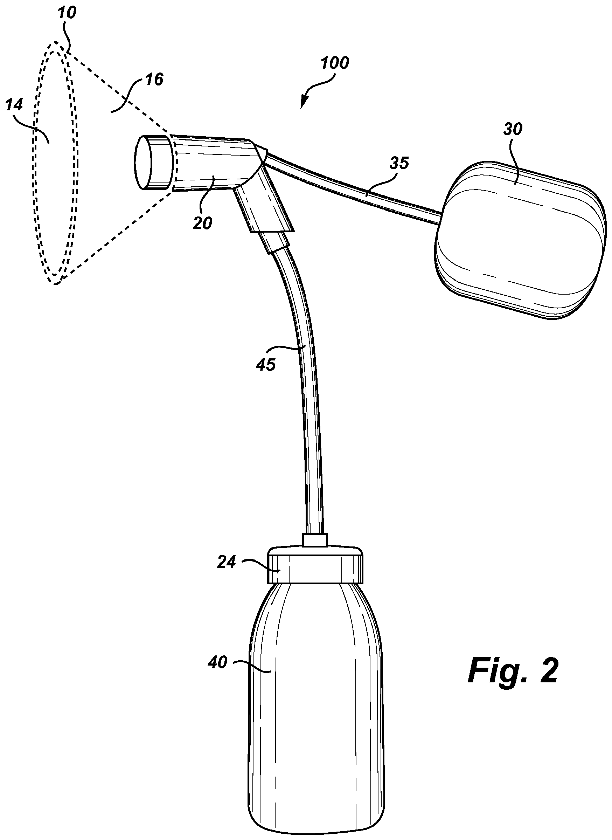

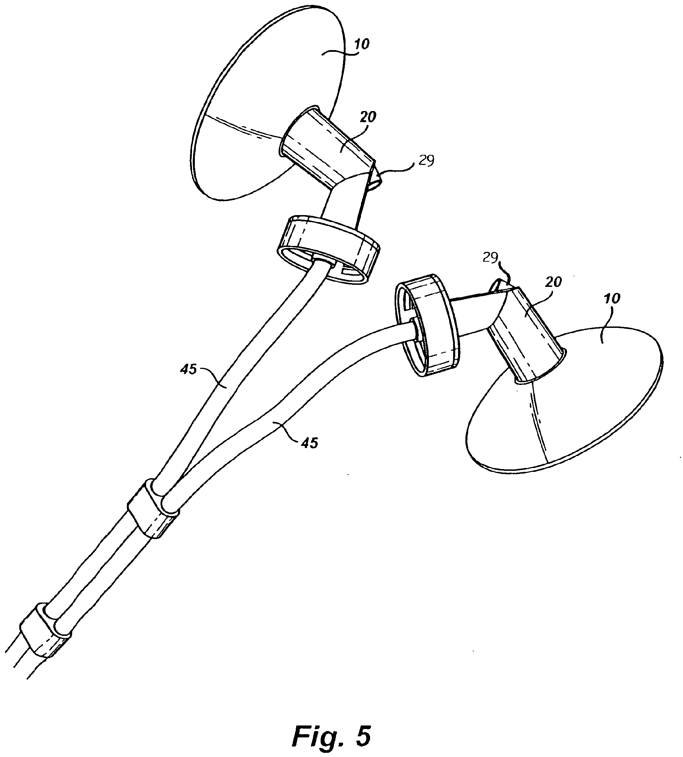

FIG. 2 illustrates other embodiments of breast pump system 100 similar to embodiments shown in Fig. 1. In Fig. 2, container 40 is coupled to adapter 20 via drain line 45. Drain line 45 may be coupled to adaptor 20 and container connector 24. Container connector 24 may contain a valve that closes when negative pressure is generated by pump 30, creating a vacuum. This valve would open when the pump cycles off the negative pressure, allowing milk to drain into container 40. Similarly, such a valve may be located on adaptor 20, or as an in-line valve in drain line 45. Drain line 45 may be long enough that container 40 may be supported by something other than adapter 20. For example, container 40 may rest on a table or chair while a woman is expressing milk, or may be held on a belt or other supporting structure. Drain line 45 may be any device capable of transferring milk from adapter 20 to container 40. For example, drain line 45 may be plastic tubing, such as PVC tubing. Drain line 45 may be connected to adapter 20 and container 40 via any type of connection means desired. In some embodiments, such as is shown in Fig. 5, adaptor 20 may be able to connect directly to container 40, or to drain line 45, as desired.

Reducing the weight that must be supported by adapter 20 reduces the weight that must be supported by the adhesive connection of breast shield 10 to a woman's breast, and consequently, by the woman's breast. Therefore, the embodiments of Fig. 2 reduces the adhesion required in the embodiments of Fig. 1 for breast shield 10 to stay adhesively connected to a woman's breast in a hands-free manner.

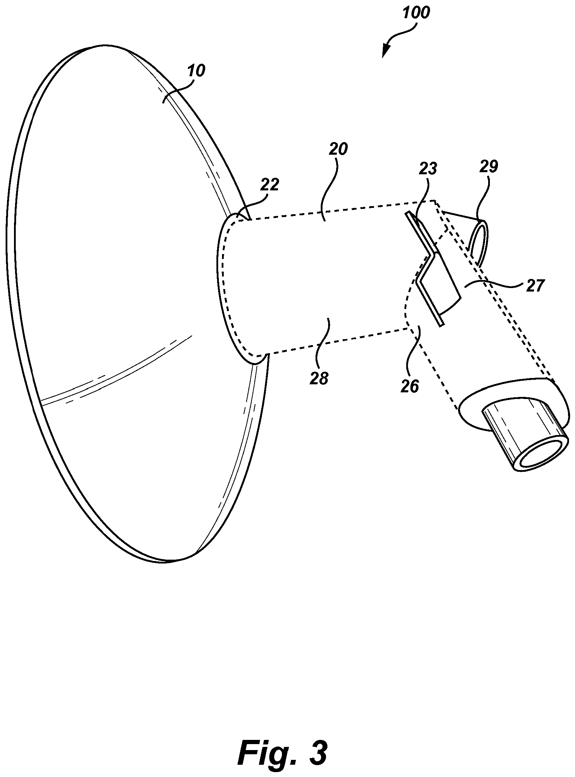

Fig. 3 illustrates the interior of and embodiment of connector 20. Connector 20 may include interior passageway 28 divided into liquid passageway 26 and vacuum passageway 27 by diverter 23. Diverter 23 may be positioned to prevent expressed milk from being sucked into pump 30. When in use, milk will be expressed into passageway 28. Diverter 23 channels the milk down liquid passageway 26, and further down by gravity into container 40. Vacuum line connector 29 is attached to pump 30, which supplies the negative pressure to express the milk.

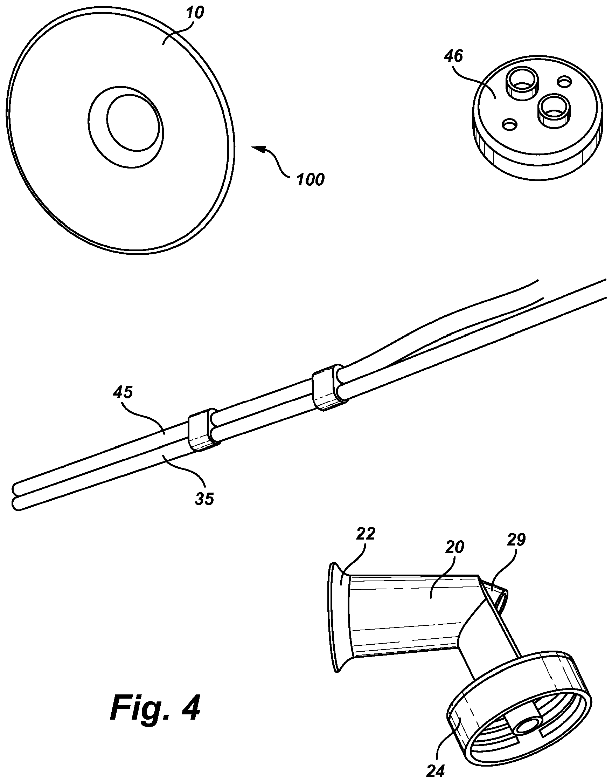

Fig. 4 illustrates components of unassembled breast pump system 100 as may be provided to an end user. System 100 may include breast shield 10, adapter 20, line connector 46, vacuum line 35, and drain line 45. Line connector 46 may be placed in the opening of a fluid storage container, such as container 40. Line connector 46 may be coupled to both vacuum line 35 and drain line 45, with vacuum line 35 going to a pump, such as pump 30, and drain line 45 going to adaptor 20. In some embodiments, vacuum line connector 29 may be capped, as the vacuum is drawn through container 40 and drain line 45, instead of directly through adaptor 20. Similarly, in some embodiments, both lines 35 and 45 may function as drain lines 45 running from dual adaptors 10 to the same container 40 through line connector 46.

Fig. 5 illustrates twin drain lines 45 connected to twin adaptors 20 and breast shields 10 that may be used to express milk from both breasts simultaneously. A single or multiple pumps may be coupled to adaptors 20 as required. Similarly, each of drain lines 45 may be connected to the same or a different container 40, and may be connected together with a "Y" connector to drain into a single bottle through a single drain line 45. Similarly, a single vacuum line from a single pump may be split with a "Y" connector to attach to both adaptors 20. It will be understood that lines 35 and 45 may be connected in any manner to their respective devices and locations, similar to as discussed with respect to vacuum line connector 29 above.

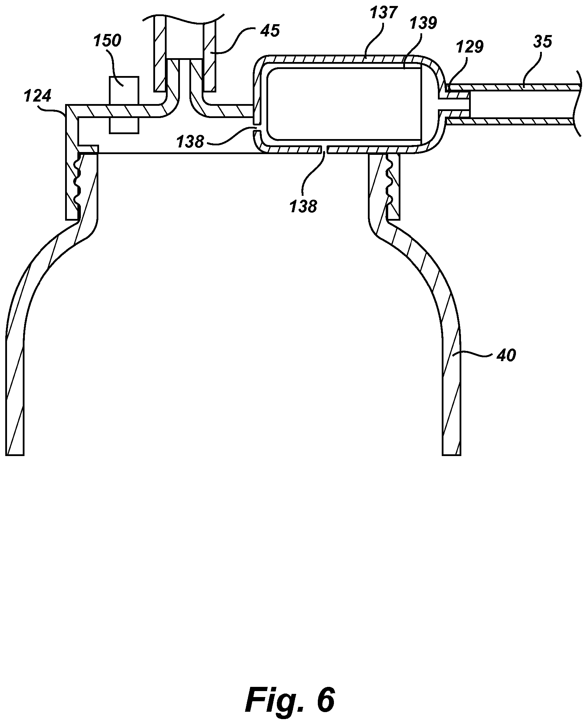

FIG. 6 illustrates a portion of an exemplary breast pump system with container connector 124. Container connector 124 may include valve 137 connected to vacuum line 35 through vacuum line connector 129. Container connector 124 may be coupled to fluid container 40 and drain line 45 similar to embodiments of connector 24 discussed above. However, container connector 124 may allow drain line 45 to both carry the expressed milk to fluid container 40, and to carry the vaccum pressure from vacuum line 35, making it possible to have only one connection to adaptor 20, as previously described.

Valve 137 may include collapsible bladder 139, which may collapse as a vacuum is drawn from vacuum line 35, thus producing a pressure drop in fluid container 40, drain line 45 and adaptor 20 sufficient to cause milk from a lactating woman's breast to be expressed. The expressed milk may then be drawn down drain line 45 into fluid container 40. Valve 137 may also include air passageways 138 in communication with the interior of fluid container 40.

Container connector 124 may be connected to fluid container 40 with a threaded connection, similar to the connection of connector 24 to fluid container 40 described above. Burp valve 150 may provide for the expulsion of excess pressure from fluid container 40 as milk collects in container 40 to allow valve 137 to continue to provide negative pressure to fluid container 40 and drain line 45.

In some embodiments, valve 137 may be an in-line valve placed in vacuum line 35, and may be constructed in any manner that allows a vacuum to be drawn in drain line 45 while eliminating the possibility of fluid from travelling from fluid container 40 through vacuum line 35 an into pump 30. In some embodiments, valve 137 may not be needed, depending on the configuration of the various parts and components of the breast pump system.

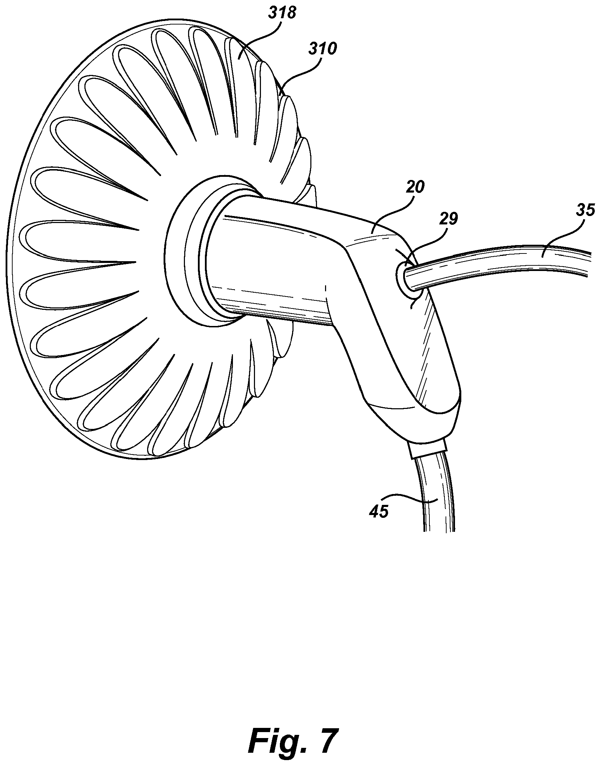

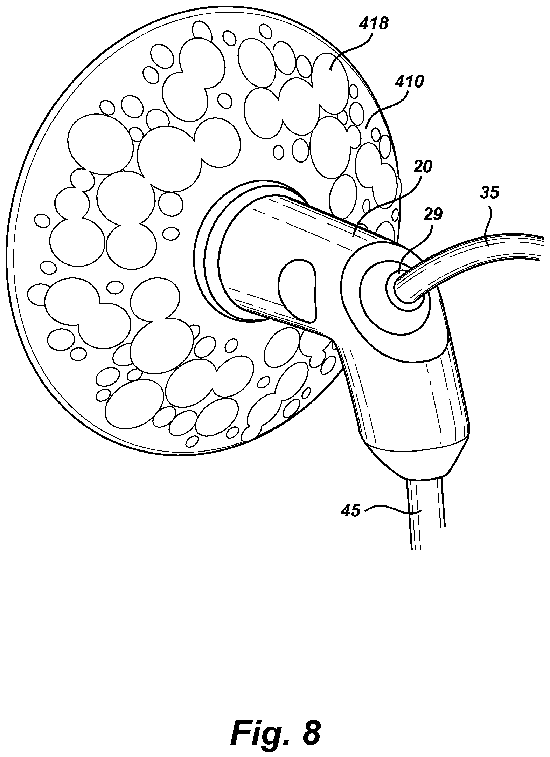

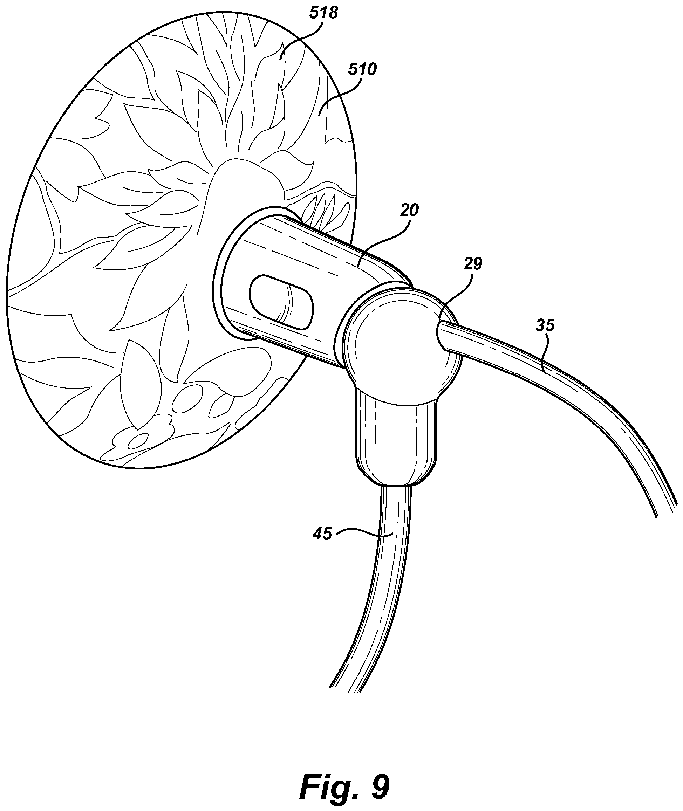

FIGS. 7 through 9 illustrate exemplary embodiments of breast shield 10 of Figs. 1-5. Each of breast shields 310, 410, 510 includes surface features 318, 418, 518, respectively. Breast shield 310 includes surface features 318 resembling flower pedals extending outwardly from adaptor 20. Similarly, breast shield 410 includes surface features 418 resembling bubbles, and breast shield 510 includes surface features 518 resembling leaves or other nature-styled images. Surface features 318, 418, 518 may provide structure, and may provide additional adhesion for inner surface 14. Similarly, as shown in the Figures, adaptor 20 may be provided in a number of different profiles and designs.

It should be understood the disclosed embodiments of the disclosed embodiments of breast pump systems are exemplary only and do not limit the breadth of the disclosure. Likewise, it should be understood that the shape, material, edge design, and surface area of the illustrated embodiments are only exemplary of embodiments of breast shields and are not limiting, as breast shields falling within the scope of the appended claims may have different shapes, edge profiles, etc., while performing the same function.

As will be apparent to those skilled in the art in which the invention is addressed, the present invention may be embodied in forms other than those specifically disclosed above without departing from the scope of the appended claims. Particular embodiments of the present invention described above are therefore to be considered in all respects as illustrative and not restrictive. The scope of the present invention is as set forth in the appended claims rather than being limited to the example contained in the foregoing description.

## Claims

1. A hands-free breast pump device, comprising:
a breast shield (10, 310, 410, 510) having an inner surface configured to form a seal with a lactating woman's breast; and
an adaptor (20) configured to couple the breast shield (10, 310, 410, 510) to a breast pump system;
a fluid container (40) for receiving breast milk;
an elongate flexible drain line (45) extending between the adaptor (20) and the fluid container (40) and separating the breast shield (10, 310, 410, 510) and adaptor (20) from the fluid container (40), wherein the fluid container is moveable independent of the breast shield (10, 310, 410, 510) and the adaptor (20);
wherein an inner surface (14) of the breast shield (10, 310, 410, 510) comprises an adhesive material that is adapted to adhesively attach to the breast surrounding the areola without preventing expression of milk from the nipple through the opening of the breast shield (10, 310, 410, 510), and that is sufficiently adhesive to adhere the breast shield (10, 310, 410, 510) to skin and support the breast shield (10, 310, 410, 510) and adaptor (20) during the duration required to express a desired amount of milk independent of other attachment mechanisms.

2. The device of claim 1, wherein the breast shield (10, 310, 410, 510) is formed of a soft, flexible material.

3. The device of claim 2, wherein the material is selected from silicone rubber, Styrene-Ethylene-Butylene-Styrene (SEBS), Styrene-Ethylene-Propylene-Styrene (SEPS), and Styrene-Ethylene-Ethylene-Propylene-Styrene (SEEPS) copolymers.

4. The device of claim 1, wherein the adaptor (20) includes a passageway (26, 28) formed therein in fluid communication with a fluid container.

5. The device of claim 4, wherein the adaptor (20) further includes a passageway (27, 28) in fluid communication with a pump (30).

6. The device of claim 5, wherein the adaptor (20) further includes a diverter (23) configured to prevent fluid from flowing into the pump (30).

7. The device of claim 1, wherein the breast shield (10, 310, 410, 510) includes surface features (318, 418, 518) configured to provide rigidity or to provide additional adhesion.

8. A breast pump system comprising the device of claim 1, wherein the fluid container (40) is coupled to the adaptor (20) and configured such that milk expressed flows through the adaptor (20) and into the fluid container (40), and wherein the breast shield (10, 310, 410, 510) is configured to adhesively hold the adaptor (20) in place during the expression of milk from a woman's breast.

9. The device of claim 1, wherein the adaptor (20) includes a sleeve (22) with an end for forming a seal against or around the areola of a woman's breast.

10. The breast pump system of claim 1, wherein the breast shield (10, 310, 410, 510) is configured to adhere without application of separate adhesives to either the breast shield or a woman's breast.

11. The device of claim 1, wherein the breast shield comprises an opening (12); and
wherein the adaptor (20) is configured to mate with said breast shield via the opening (12) in the breast shield (10, 310, 410, 510); and
wherein the device further comprises a fluid container (40) coupled to the adaptor (20) and configured such that milk expressed flows through the adaptor (20) and into the fluid container (40).

12. The device of claim 1, wherein the breast shield (10, 310, 410, 510) is invertible to provide enhanced adherence when placed on a woman's breast.

13. A method of hands free collection of milk, the method comprising:
selecting a breast shield (10, 310, 410, 510) having an inner surface configured to form a seal with a lactating woman's breast and adapted to adhesively attach to the breast; an adaptor (20) configured to couple the breast shield (10, 310, 410, 510) to a breast pump system via an elongate flexible drain line (45) extending between the adaptor (20) and a fluid container (40) and separating the breast shield (10, 310, 410, 510) and adaptor (20) from the fluid container (40), such that the fluid container is moveable independent of the breast shield (10, 310, 410, 510) and the adaptor (20); and
wherein the method includes adhesively attaching the breast shield (10, 310, 410, 510) to a breast with the adhesive inner surface and without the use of straps by adhesively attaching the inner surface of the breast shield (10, 310, 410, 510) to the breast, via an adhesive material of the inner surface (14), surrounding the areola without preventing expression of milk from the nipple through the opening of the breast shield (10, 310, 410, 510) and such that the breast shield (10, 310, 410, 510) adheres to the skin and supports the breast shield (10, 310, 410, 510) and adaptor (20) during the duration required to express a desired amount of milk independent of other attachment mechanisms.

14. The method of claim 13, wherein the breast shield (10, 310, 410, 510) is connected to the adaptor (20) and the adaptor (20) is attached to the flexible drain line (45) and wherein the method comprises attaching the breast shield (10, 310, 410, 510) to a breast such that the adhesive inner surface holds the breast shield (10, 310, 410, 510), the adaptor (20) and the flexible drain line (45) supported from the breast.

15. The method of claim 14, wherein the method further comprises connecting the adaptor (20) to the container (40) by an elongate tubing.

16. The method of claim 14, wherein the method further comprises connecting the adaptor (20) to a pump (30).

## Patentansprüche

1. Freihand-Milchpumpenvorrichtung, umfassend:
ein Brustschild (10, 310, 410, 510) mit einer Innenfläche, die so ausgebildet ist, dass sie eine Dichtung mit der Brust einer stillenden Frau bildet; und
einen Adapter (20), der so ausgebildet ist, dass er den Brustschild (10, 310, 410, 510) an ein Milchpumpensystem koppelt;
einen Flüssigkeitsbehälter (40) zur Aufnahme von Muttermilch;
eine längliche flexible Ablaufleitung (45), die sich zwischen dem Adapter (20) und dem Flüssigkeitsbehälter (40) erstreckt und den Brustschild (10, 310, 410, 510) und den Adapter (20) von dem Flüssigkeitsbehälter (40) trennt, wobei der Flüssigkeitsbehälter unabhängig von dem Brustschild (10, 310, 410, 510) und dem Adapter (20) beweglich ist;
wobei eine Innenfläche (14) des Brustschildes (10, 310, 410, 510) ein Klebematerial umfasst, das so beschaffen ist, dass es an der den Warzenhof umgebenden Brust haftet, ohne das Ausdrücken von Milch aus der Brustwarze durch die Öffnung des Brustschildes (10, 310, 410, 510) zu verhindern, und das ausreichend klebend ist, um das Brustschild (10, 310, 410, 510) an der Haut anzukleben und das Brustschild (10, 310, 410, 510) und den Adapter (20) unabhängig von anderen Befestigungsmechanismen während der Dauer zu halten, die erforderlich ist, um eine gewünschte Milchmenge auszudrücken.

2. Vorrichtung nach Anspruch 1, wobei der Brustschild (10, 310, 410, 510) aus einem weichen, flexiblen Material gebildet ist.

3. Vorrichtung nach Anspruch 2, wobei das Material ausgewählt ist aus Silikonkautschuk, Styrol-Ethylen-Butylen-Styrol (SEBS), Styrol-Ethylen-Propylen-Styrol (SEPS) und Styrol-Ethylen-Ethylen-Propylen-Styrol (SEEPS) Copolymeren.

4. Vorrichtung nach Anspruch 1, wobei der Adapter (20) einen darin ausgebildeten Durchgang (26, 28) aufweist, der in Fluidverbindung mit einem Flüssigkeitsbehälter steht.

5. Vorrichtung nach Anspruch 4, wobei der Adapter (20) ferner einen Durchgang (27, 28) aufweist, der in Fluidverbindung mit einer Pumpe (30) steht.

6. Vorrichtung nach Anspruch 5, wobei der Adapter (20) ferner eine Umlenkeinrichtung (23) aufweist, die so ausgebildet ist, dass sie verhindert, dass Flüssigkeit in die Pumpe (30) fließt.

7. Vorrichtung nach Anspruch 1, wobei der Brustschild (10, 310, 410, 510) Oberflächenmerkmale (318, 418, 518) aufweist, die so ausgebildet sind, dass sie für Steifigkeit sorgen oder zusätzliche Haftung bieten.

8. Milchpumpensystem, das die Vorrichtung nach Anspruch 1 umfasst, wobei der Flüssigkeitsbehälter (40) mit dem Adapter (20) gekoppelt und so ausgebildet ist, dass die ausgedrückte Milch durch den Adapter (20) und in den Flüssigkeitsbehälter (40) fließt, und wobei der Brustschild (10, 310, 410, 510) so ausgebildet ist, dass er den Adapter (20) während des Ausdrückens von Milch aus der Brust einer Frau klebend an seinem Platz hält.

9. Vorrichtung nach Anspruch 1, wobei der Adapter (20) eine Hülse (22) mit einem Ende zum Bilden einer Dichtung gegen oder um den Warzenhof der Brust einer Frau umfasst.

10. Milchpumpensystem nach Anspruch 1, wobei der Brustschild (10, 310, 410, 510) so ausgebildet ist, dass er ohne Anwendung separater Klebstoffe auf entweder den Brustschild oder die Brust einer Frau haftet.

11. Vorrichtung nach Anspruch 1, wobei der Brustschild eine Öffnung (12) aufweist; und
wobei der Adapter (20) so ausgebildet ist, dass er mit dem Brustschild über die Öffnung (12) im Brustschild (10, 310, 410, 510) zusammenpasst; und
wobei die Vorrichtung ferner einen Flüssigkeitsbehälter (40) umfasst, der mit dem Adapter (20) verbunden und so ausgebildet ist, dass ausgedrückte Milch durch den Adapter (20) und in den Flüssigkeitsbehälter (40) fließt.

12. Vorrichtung nach Anspruch 1, wobei der Brustschild (10, 310, 410, 510) umkehrbar ist, um eine verbesserte Haftung zu bieten, wenn er auf die Brust einer Frau gelegt wird.

13. Verfahren zum freihändigen Sammeln von Milch, wobei das Verfahren umfasst:
Auswählen eines Brustschildes (10, 310, 410, 510) mit einer Innenfläche, die so ausgebildet ist, dass sie eine Dichtung mit der Brust einer stillenden Frau bildet, und so angepasst ist, dass sie an der Brust haftet; einen Adapter (20), der so ausgebildet ist, dass er den Brustschild (10, 310, 410, 510) über eine längliche, flexible Ablaufleitung (45), die sich zwischen dem Adapter (20) und einem Flüssigkeitsbehälter (40) erstreckt und den Brustschild (10, 310, 410, 510) und den Adapter (20) von dem Flüssigkeitsbehälter (40) trennt, mit einem Milchpumpensystem koppelt, so dass der Flüssigkeitsbehälter unabhängig von dem Brustschild (10, 310, 410, 510) und dem Adapter (20) beweglich ist; und
wobei das Verfahren das klebende Befestigen des Brustschildes (10, 310, 410, 510) an einer Brust mit der klebenden Innenfläche und ohne die Verwendung von Bändern durch klebendes Befestigen der Innenfläche des Brustschildes (10, 310, 410, 510) an der Brust über ein klebendes Material der Innenfläche (14) umfasst, die den Warzenhof umgibt, ohne das Ausdrücken von Milch aus der Brustwarze durch die Öffnung des Brustschildes (10, 310, 410, 510) zu verhindern, und so, dass der Brustschild (10, 310, 410, 510) an der Haut haftet und den Brustschild (10, 310, 410, 510) und den Adapter (20) während der Dauer, die erforderlich ist, um eine gewünschte Milchmenge auszudrücken, unabhängig von anderen Befestigungsmechanismen stützt.

14. Verfahren nach Anspruch 13, wobei der Brustschild (10, 310, 410, 510) mit dem Adapter (20) verbunden ist und der Adapter (20) an der flexiblen Abflussleitung (45) angebracht ist und wobei das Verfahren das Anbringen des Brustschilds (10, 310, 410, 510) an einer Brust umfasst, so dass die klebende Innenfläche den Brustschild (10, 310, 410, 510), den Adapter (20) und die flexible Abflussleitung (45) von der Brust gestützt hält.

15. Verfahren nach Anspruch 14, wobei das Verfahren ferner das Verbinden des Adapters (20) mit dem Behälter (40) durch einen langgestreckten Schlauch umfasst.

16. Verfahren nach Anspruch 14, wobei das Verfahren ferner das Verbinden des Adapters (20) mit einer Pumpe (30) umfasst.

## Revendications

1. Dispositif de tire-lait mains libres, comprenant :
un bouclier mammaire (10, 310, 410, 510) ayant une surface interne configurée pour former un joint avec le sein d'une femme allaitante ; et
un adaptateur (20) configuré pour coupler le bouclier mammaire (10, 310, 410, 510) à un système de tire-lait ;
un récipient de fluide (40) pour recevoir le lait maternel ;
une conduite de drainage flexible allongée (45) s'étendant entre l'adaptateur (20) et le récipient de fluide (40) et séparant le bouclier mammaire (10, 310, 410, 510) et l'adaptateur (20) du récipient de fluide (40), dans lequel le récipient de fluide est mobile indépendamment du bouclier mammaire (10, 310, 410, 510) et de l'adaptateur (20) ;
dans lequel une surface intérieure (14) du bouclier mammaire (10, 310, 410, 510) comprend un matériau adhésif qui est adapté pour se fixer de manière adhésive au sein entourant l'aréole sans empêcher l'expression du lait du mamelon à travers l'ouverture du bouclier mammaire (10, 310, 410, 510), et qui est suffisamment adhésif pour faire adhérer le bouclier mammaire (10, 310, 410, 510) à la peau et supporter le bouclier mammaire (10, 310, 410, 510) et l'adaptateur (20) pendant la durée requise pour exprimer une quantité souhaitée de lait indépendamment d'autres mécanismes de fixation.

2. Dispositif selon la revendication 1, dans lequel le bouclier mammaire (10, 310, 410, 510) est formé d'un matériau souple et flexible.

3. Dispositif selon la revendication 2, dans lequel le matériau est choisi parmi le caoutchouc de silicone, le styrène-éthylène-butylène-styrène (SEBS), le styrène-éthylène-propylène-styrène (SEPS) et les copolymères styrène-éthylène-éthylène-propylène-styrène (SEEPS).

4. Dispositif selon la revendication 1, dans lequel l'adaptateur (20) comprend un passage (26, 28) formé dans celui-ci en communication fluidique avec un récipient de fluide.

5. Dispositif selon la revendication 4, dans lequel l'adaptateur (20) comprend en outre un passage (27, 28) en communication fluidique avec une pompe (30).

6. Le dispositif selon la revendication 5, dans lequel l'adaptateur (20) comprend en outre un déviateur (23) configuré pour empêcher le fluide de s'écouler dans la pompe (30).

7. Le dispositif selon la revendication 1, dans lequel le bouclier mammaire (10, 310, 410, 510) comprend des caractéristiques de surface (318, 418, 518) configurées pour fournir une rigidité ou pour fournir une adhérence supplémentaire.

8. Système de tire-lait comprenant le dispositif selon la revendication 1, dans lequel le récipient de fluide (40) est couplé à l'adaptateur (20) et configuré de telle sorte que le lait exprimé s'écoule à travers l'adaptateur (20) et dans le récipient de fluide (40), et dans lequel le bouclier mammaire (10, 310, 410, 510) est configurée pour maintenir de manière adhésive l'adaptateur (20) en place pendant l'expression du lait du sein d'une femme.

9. Dispositif selon la revendication 1, dans lequel l'adaptateur (20) comprend un manchon (22) avec une extrémité pour former un joint contre ou autour de l'aréole du sein d'une femme.

10. Le système de tire-lait selon la revendication 1, dans lequel le bouclier mammaire (10, 310, 410, 510) est configuré pour adhérer sans application d'adhésifs séparés soit au bouclier mammaire, soit au sein d'une femme.

11. Dispositif selon la revendication 1, dans lequel le bouclier mammaire comprend une ouverture (12) ; et
dans lequel l'adaptateur (20) est configuré pour s'adapter audit bouclier mammaire via l'ouverture (12) dans le bouclier mammaire (10, 310, 410, 510) ; et
dans lequel le dispositif comprend en outre un récipient de fluide (40) couplé à l'adaptateur (20) et configuré de telle sorte que le lait exprimé s'écoule à travers l'adaptateur (20) et dans le récipient de fluide (40).

12. Dispositif selon la revendication 1, dans lequel le bouclier mammaire (10, 310, 410, 510) est réversible pour assurer une meilleure adhérence lorsqu'il est placé sur le sein d'une femme.

13. Procédé de collecte de lait en mains libres, le procédé comprenant :
la sélection d'un bouclier mammaire (10, 310, 410, 510) ayant une surface interne configurée pour former un joint avec le sein d'une femme allaitante et adaptée pour se fixer de manière adhésive au sein ; un adaptateur (20) configuré pour coupler le bouclier mammaire (10, 310, 410, 510) à un système de tire-lait par l'intermédiaire d'une conduite de drainage flexible allongée (45) s'étendant entre l'adaptateur (20) et un récipient de fluide (40) et séparant le bouclier mammaire (10, 310, 410, 510) et l'adaptateur (20) du récipient de fluide (40), de sorte que le récipient de fluide est mobile indépendamment du bouclier mammaire (10, 310, 410, 510) et de l'adaptateur (20) ; et
dans lequel le procédé comprend la fixation par adhésif du bouclier mammaire (10, 310, 410, 510) à un sein avec la surface interne adhésive et sans l'utilisation de sangles en fixant par adhésif la surface interne du bouclier mammaire (10, 310, 410, 510) au sein, via un matériau adhésif de la surface interne (14), entourant l'aréole sans empêcher l'expression du lait du mamelon à travers l'ouverture du bouclier mammaire (10, 310, 410, 510) et de telle sorte que le bouclier mammaire (10, 310, 410, 510) adhère à la peau et supporte le bouclier mammaire (10, 310, 410, 510) et l'adaptateur (20) pendant la durée nécessaire pour exprimer une quantité désirée de lait indépendamment d'autres mécanismes de fixation.

14. Procédé selon la revendication 13, dans lequel le bouclier mammaire (10, 310, 410, 510) est connecté à l'adaptateur (20) et l'adaptateur (20) est fixé à la conduite de drainage flexible (45) et dans lequel le procédé comprend la fixation du bouclier mammaire (10, 310, 410, 510) à un sein de sorte que la surface interne adhésive maintienne le bouclier mammaire (10, 310, 410, 510), l'adaptateur (20) et la conduite de drainage flexible (45) supportés par le sein.

15. Procédé selon la revendication 14, dans lequel le procédé comprend en outre la connexion de l'adaptateur (20) au récipient (40) par un tube allongé.

16. Procédé selon la revendication 14, dans lequel le procédé comprend en outre la connexion de l'adaptateur (20) à une pompe (30).
